# DEMANDE DE BREVET EUROPEEN

(11) **EP 1 025 814 A1**
(43) Date de publication de la demande: **09.08.2000**
(21) Numéro de dépôt: 00400142.6
(22) Date de dépôt: 20.01.2000
(51) Int. Cl.: A61F 2/32, A61L 27/10

(54) **Prothèse fémorale céramique/céramique de petit diamètre.**

(30) Priorité: 04.02.1999 FR 9901315
(71) Demandeur: NORTON DESMARQUEST FINE CERAMICS, 92400 Courbevoie (FR)
(72) Inventeur: Cales, Bernard, 27000 Evreux (FR); Drouin, Jean-Michel, 78600 Maisons Laffitte (FR); Stefani, Yves, 92170 Vanves (FR)
(74) Mandataire: Rinuy, Santarelli

(57) **Abrégé**

Une prothèse fémorale (10) comporte une tête fémorale (11) adaptée à être emmanchée sur une tige fémorale (12) destinée à être solidarisée à un fémur d'un patient, et une cupule (13) destinée à être solidarisée au bassin (14) de ce patient, la tête (11) étant montée en sorte de pouvoir glisser en rotation dans une cavité ménagée à l'intérieur de la cupule (13), cette tête fémorale (11) étant au moins dans sa partie extérieure réalisée en un matériau céramique et ayant une surface extérieure sphérique de diamètre sensiblement égal à 22mm, et cette cupule (13) étant au moins dans sa partie interne réalisée en un matériau céramique et ayant une surface interne sphérique de diamètre sensiblement égal à 22mm.

## Description

L'invention concerne une prothèse fémorale de petit diamètre.

Ainsi qu'on le sait, une prothèse fémorale comporte :
- une tête destinée à être engagée sur une tige solidaire d'un fémur ,
- et une cupule destinée à être solidarisée au bassin de l'utilisateur, et comportant une cavité destinée a recevoir pour des mouvement de frottement en rotation la tête fémorale.

L' invention a pour objet une prothèse fémorale, comportant une tête fémorale d'une part et une cupule d'autre part, dont la combinaison puisse se faire selon une surface sphérique de l'ordre de 22,22 mm, tout en atteignant une résistance à la rupture au moins égale à 46 kN.

L'invention propose à cet effet une prothèse fémorale comportant une tête fémorale adaptée à être emmanchée sur une tige fémorale destinée à être solidarisée à un fémur d'un patient, et une cupule destinée à être solidarisée au bassin de ce patient, la tête étant montée en sorte de pouvoir glisser en rotation dans une cavité ménagée à l'intérieur de la cupule, cette tête fémorale étant au moins dans sa partie extérieure réalisée en un matériau céramique et ayant une surface extérieure sphérique de diamètre sensiblement égal à 22 mm, et cette cupule étant au moins dans sa partie interne réalisée en un matériau céramique et ayant une surface interne sphérique de diamètre sensiblement égal à 22 mm.

Selon des dispositions avantageuses de l'invention, avantageusement combinées :
- le diamètre de la tête est sensiblement égal à 22,22 mm.
- le diamètre de la surface interne de la cupule est supérieur à celui de la surface extérieure de la tête fémorale.
- la tête fémorale comporte au moins 95% de zircone.
- cette zircone est de la zircone tétragonale polycristalline stabilisée à l'oxyde d'yttrium.
- cette tête a une densité d'au moins 98% de la densité maximale théorique et est réalisée par frittage isostatique haute pression.
- la zircone a une taille moyenne de grain au plus égale à un micron.
- la zircone a un module de flexion 3-points supérieur à 920 Mpa.
- le diamètre d'entrée de la cavité de la tête est compris entre 7 et 16 mm.
- la cupule comporte un insert céramique à l'intérieur d'une coiffe extérieure.

Selon une première option possible, l'insert est solidarisé à la coiffe extérieure par une coiffe intermédiaire, laquelle est avantageusement réalisée en polyéthyléne.

Selon une seconde option possible, l'insert céramique est engagé à force dans la coiffe extérieure, par emmanchement conique. Cet emmanchement conique a de préférence une conicité comprise entre 4 et 25° ; elle est par exemple de 20° environ.

Le matériau dont la cupule est formée au moins dans sa portion interne est avantageusement une alumine. Toutefois, d'autres céramiques sont possibles, notamment parmi les oxydes.

En pratique les matériaux utilisés sont des matériaux de grade biomédical, respectant les normes en vigueur, qu'il s'agisse de la zircone ou de l'alumine. Les matériaux utilisés sont en effet des matériaux connus en soi, a titre isolé, dans le domaine des prothèses ou des implants.

De manière préférée, la tête coopère avec une tige fémorale de type 12/14, avec une partie tronconique délimitée par des cercles extrêmes de diamètres de l'ordre de 12 et 14 mm respectivement. Celle coopération est avantageusement conforme aux enseignements du document WO98/14141.

Des objets, caractéristiques et avantages de l'invention ressortent de la description donnée ci dessous, en regard du dessin annexé sur lequel:
- la figure 1 est une vue schématique en demi coupe axiale, d'une prothèse fémorale selon un premier mode de réalisation de l'invention,
- la figure 2 est une demi-vue axiale d'une autre prothèse fémorale selon un second mode de réalisation de l'invention,
- la figure 3 montre de manière isolée une demi vue en coupe axiale de l'insert de la figure 1, et
- la figure 4 est une vue en demi coupe axiale de l'insert de la figure 2.

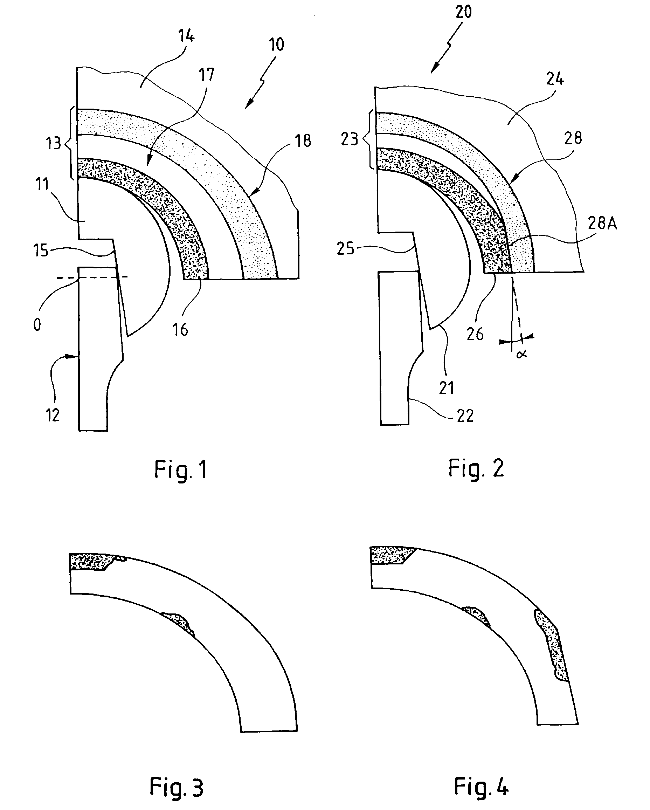

Les figures 1 et 2 représentent deux modes de réalisation d'une prothèse fémorale conforme à l'invention.

Dans le mode de réalisation de la figure 1, la prothèse fémorale, désignée sous la référence générale 10, comporte une tête 11, engagée sur une tige 12, ainsi qu'une cupule 13 solidaire d'un socle schématisé en 14.

La tige 12 est une tige fémorale , en ce sens que, en son extrémité inférieure, elle est destinée à être engagée à force dans l'extrémité supérieure du fémur d'un patient destiné à recevoir une telle prothèse fémorale.

Une telle tige fémorale est classiquement métallique, par exemple réalisée en alliage de titane, en alliage chrome-cobalt, ou en un alliage de type inox.

Celle tige comporte, en sa partie supérieure une portion tronconique qui rentre dans une cavité également tronconique ménagée dans la tête 11 ; celle cavité, repérée 15, a une conicité de préférence égale ou très légèrement supérieure a la conicité de la partie supérieure de la tige.

On remarque en outre que celle tige, engagée normalement à force dans la cavité 15 de la tête 11, ne pénètre pas jusqu'au fond de la cavité, mais reste en deçà de ce fond.

La cupule 13, dans celle exemple de la figure 1, est formée de 3 couches (mais il peut y en avoir plus).

La surface interne de celle cupule, contre laquelle vient frotter sa surface extérieure sensiblement sphérique de la tête 11 est ménagée sur un insert 16, d'épaisseur sensiblement constante.

On peut remarquer sur cette figure 1 que le diamètre interne de cette surface interne de l'insert 16 est légèrement supérieur au diamètre de la surface externe de la tête 11.

Cet insert 16 est lui même engagé dans une coiffe intermédiaire 17, d'épaisseur elle aussi, sensiblement constante.

Cette coiffe intermédiaire 17 est elle même engagée à l'intérieur d'une troisième pièce 18, ou coiffe extérieure, destinée a être solidarisée au bassin 14 d'un utilisateur.

La surface externe sphérique de la tête 11, ainsi que la surface interne sphérique de l'insert 16, ont des rayons sensiblement égaux à 22 mm (le diamètre de la tête est de préférence sensiblement égal à 22,22 mm).

Par ailleurs, la tête 11 d'une part et l'insert 16 d'autre part sont tous deux réalisés dans des matériaux céramiques.

La coiffe intermédiaire 17 est un matériau synthétique, de préférence polyéthylène à haute densité (P E H D).

Quant à la coiffe externe 18, elle est avantageusement métallique, par exemple réalisée en alliage de titane.

De manière préférée, la tête 11 est réalisée en une zircone, de préférence stabilisée à l'yttrium, de préférence réalisée par frittage isostatique haute pression.

L'insert céramique 16 est, quant à lui, avantageusement réalisé en alumine.

La zircone dont est réalisée la tête fémorale 11 a avantageusement les propriétés de la zircone vendue sous la marque commerciale "PROZYR" ® telle que vendue par la société NORTON DESMARQUEST.

En ce qui concerne l'insert 16 de la cupule, l'alumine qui le constitue est avantageusement un matériau ayant des propriétés proches de l'alumine vendue sous la marque INCERAL ® vendue également par NORTON DESMARQUEST.

Il est apparu de manière tout a fait surprenante que la combinaison de la résistance mécanique de la zircone surtout lorsqu'il s'agit d'une zircone de type PROZYR, et de l'usure minimale du couple des matériaux zircone (de préférence PROZYR) et alumine (de préférence INCERAL) offre une solution optimale pour les couples céramique/céramique utilisés pour réaliser une prothèse fémorale type représenté figure 1.

En pratique, on peut donner à la coiffe extérieure 18 une forme géométrique complexe à choisir selon les besoins; ce n'est donc qu'à titre simplement illustratif que cette coiffe extérieure18 est représentée comme ayant une épaisseur constante en tous points.

La figure 2 représente une prothèse de hanche, schématisée sous la référence générale 20, qui comporte, comme la prothèse 10 de la figure 1, une tête 21, engagée sur une tige fémorale 22, et engagée à frottement dans une cupule 23 solidaire d'un socle 24, par exemple constitué par le bassin d'un patient.

Les indications qui précédent, données à propos de la tige fémorale et de la tête fémorale sont également valables pour la prothèse fémorale 20 de la figure 2.

En fait, la différence essentielle entre la prothèse fémorale 20 et celle qui a été décrite à propose de la figure 1, réside dans le fait que la cupule comporte 2 matériaux uniquement; il 'y a pas de coiffe intermédiaire, par exemple en polyéthylène.

Dans le cas de celle prothèse 20, la solidarisation entre l'insert 26 d'une part et la coiffe externe 28 d'autre part est de préférence réalisée par un emmanchement à force de type conique. On observe ainsi, sur cette figure 2, que, à la différence de la figure 1 où la surface externe de l'insert céramique est représentée comme étant sphérique, la surface externe de l'insert 26 de cette figure 2 comporte en partie basse, donc en direction du fémur, une portion tronconique évasée en direction de ce fémur.

Celle portion tronconique, repérée 28A, se raccorde au reste de la surface extérieure de l'insert 28, qui peut être sphérique.

Bien entendu, la surface extérieure de l'insert 16 de la figure 1 présente comme cela vient d'être précisé une surface extérieure sphérique ; toutefois elle peut avoir d'autres formes, y compris une forme du type de la surface externe qui vient d'être décrite a propos de cette figure 2.

Comme précédemment, la tête fémorale est avantageusement réalisée en zircone, de préférence de type PROZYR, tandis que l'insert céramique 26 est avantageusement réalise en alumine, de préférence de type INCERAL .

Ces matériaux, connus en soi, ont des qualités chirurgicales et sont définis par les normes qu'ils satisfont.

Les figures 3 et 4 représentent, de manière isolée, l'insert céramique des figures 1 et 2 respectivement.

Sur ces figures des zones foncées visualisent les endroits de ces inserts où les contraintes de tension sont maximales.

Sur la figure 3, une zone de contrainte maximum est observée au sommet de l'insert, du coté externe. En outre, une zone de forte contrainte est observée du coté interne de l'insert, sensiblement à 45 degrés par rapport au centre de cette surface interne.

Sur la figure 4, on observe 3 zones de contrainte importante, à savoir, au sommet de l'insert du coté supérieur, du coté interne de l'insert sensiblement à 45 degrés par rapport au centre de la surface interne et enfin en la zone la plus profonde en laquelle se fait l'emmanchement conique.

A titre d exemple, pour une épaisseur d'insert de l'ordre de 4 mm, et un angle de 20 degrés dans la cas de l'emmanchement conique, les efforts constatés peuvent atteindre 185 MPa au sommet , sur la figure 4 , tandis qu'elle n'est que de l'ordre de 70 MPa dans la zone proche de l'emmanchement conique. Les valeurs constatées pour les zones figure 2 sont situées entre ces extrêmes.

Le matériau choisi avait une contrainte à la rupture en flexion ―3 points de 600 MPa.

L'angle de 20 degrés qui vient d'être mentionné a propos de l'emmanchement conique entre l'insert céramique et la coiffe extérieurs peut, selon les cas, être augmenté ou diminué à l'intérieur d'une plage angulaire dont les seuils sont avantageusement de l'ordre de 4 et 25 degrés.

## Revendications

1. Prothèse fémorale comportant une tête fémorale (11, 21) adaptée à être emmanchée sur une tige fémorale (12, 22) destinée à être solidarisée à un fémur d'un patient, et une cupule (13, 23) destinée à être solidarisée au bassin de ce patient, la tête étant montée en sorte de pouvoir glisser en rotation dans une cavité ménagée à l'intérieur de la cupule, cette tête fémorale étant au moins dans sa partie extérieure réalisée en un matériau céramique et ayant une surface extérieure sphérique de diamètre sensiblement égal à 22 mm, et cette cupule étant au moins dans sa partie interne réalisée en un matériau céramique et ayant une surface interne sphérique de diamètre sensiblement égal à 22 mm.

2. Prothèse fémorale selon la revendication 1, caractérisée en ce que le diamètre de la tête est sensiblement égal à 22,22 mm.

3. Prothèse fémorale selon la revendication 1 ou la revendication 2, caractérisée en ce que le diamètre de la surface interne de la cupule est supérieur à celui de la surface extérieure de la tête fémorale.

4. Prothèse fémorale selon l'une quelconque des revendications 1 à 3, caractérisée en ce que la tête fémorale comporte au moins 95% de zircone.

5. Prothèse fémorale selon la revendication 4, caractérisée en ce que cette zircone est de la zircone tétragonale polycristalline stabilisée à l'oxyde d'yttrium.

6. Prothèse fémorale selon la revendication 4 ou la revendication 5, caractérisée en ce que celle tête a une densité d'au moins 98% de la densité maximale théorique et est réalisée par frittage isostatique haute pression.

7. Prothèse fémorale selon l'une quelconque des revendications 4 à 6, caractérisée en ce que la zircone a une taille moyenne de grain au plus égale à un micron.

8. Prothèse fémorale selon l'une quelconque des revendications 4 à 7, caractérisée en ce que la zircone a un module de flexion 3-points supérieur à 920 Mpa.

9. Prothèse fémorale selon l'une quelconque des revendications 1 à 8, caractérisée en ce que le diamètre d'entrée de la cavité de la tête est compris entre 7 et 16 mm environ.

10. Prothèse fémorale selon l'une quelconque des revendications 1 à 9, caractérisée en ce que la cupule comporte un insert céramique à l'intérieur d'une coiffe extérieure.

11. Prothèse fémorale selon la revendication 10, caractérisée en ce que l'insert (16) est solidarisé à la coiffe extérieure (18) par une coiffe intermédiaire (17).

12. Prothèse fémorale selon la revendication 11, caractérisée en ce que cette coiffe intermédiaire (17) est réalisée en polyéthylène.

13. Prothèse fémorale selon la revendication 10, caractérisée en ce que l'insert (26) céramique est engagé à force dans la coiffe extérieure (28), par emmanchement conique.

14. Prothèse fémorale selon la revendication 13, caractérisée en ce que l'emmanchement conique a une conicité comprise entre 4° et 25°.

15. Prothèse fémorale selon la revendication 14, caractérisée en ce que la conicité est de 20° environ.

16. Prothèse fémorale selon l'une quelconque des revendications 1 à 15, caractérisée en ce que le matériau dont la cupule est formée au moins dans sa portion interne est une alumine.
